# EUROPEAN PATENT SPECIFICATION

(11) **EP 3 161 482 B1**
(45) Date of publication and mention of the grant of the patent: **26.08.2020**
(21) Application number: 15811137.7
(22) Date of filing: 26.06.2015
(51) Int. Cl.: G01N 33/68

(54) **METHOD FOR ENRICHING CNS-DERIVED EXOSOMES**
VERFAHREN ZUR ANREICHERUNG VON EXOSOMEN AUS DEM ZNS
PROCÉDÉ POUR ENRICHIR DES EXOSOMES DÉRIVÉS DU SYSTÈME NERVEUX CENTRAL

(30) Priority: 27.06.2014 US 201462018081 P
(43) Date of publication of application: 03.05.2017
(73) Proprietor: XY Evergreen Technology Company, Beijing (CN)
(72) Inventor: ZHANG, Jing, Mercer Island, Washington 98040 (US)
(74) Representative: Zimmermann, Tankred Klaus
(86) International application number: PCT/US2015/038108
(87) International publication number: WO 2015/200851

(56) References cited:
- WO-A1-2014/059052
- US-A1- 2008 241 924
- US-A1- 2008 241 924
- US-A1- 2010 184 046
- US-A1- 2010 184 046
- US-A1- 2015 119 278
- M. W. GRANER ET AL: "Proteomic and immunologic analyses of brain tumor exosomes", THE FASEB JOURNAL, vol. 23, no. 5, 24 December 2008 (2008-12-24), pages 1541-1557, XP055416153, US ISSN: 0892-6638, DOI: 10.1096/fj.08-122184
- MIN SHI ET AL: "Plasma exosomal [alpha]-synuclein is likely CNS-derived and increased in Parkinson's disease", ACTA NEUROPATHOLOGICA., vol. 128, no. 5, 6 July 2014 (2014-07-06), pages 639-650, XP055225837, BERLIN, DE ISSN: 0001-6322, DOI: 10.1007/s00401-014-1314-y
- S. SAMAN ET AL: "Exosome-associated Tau Is Secreted in Tauopathy Models and Is Selectively Phosphorylated in Cerebrospinal Fluid in Early Alzheimer Disease", JOURNAL OF BIOLOGICAL CHEMISTRY, vol. 287, no. 6, 4 November 2011 (2011-11-04), pages 3842-3849, XP055089563, ISSN: 0021-9258, DOI: 10.1074/jbc.M111.277061
- KENNETH W WITWER ET AL: "Standardization of sample collection,isolation and analysis methods in extracellular vesicle research", JOURNAL OF EXTRACELLULAR VESI, COACTION PUBLISHING, SE, vol. 2, 27 May 2013 (2013-05-27), page 25pp, XP007923200, ISSN: 2001-3078, DOI: 10.3402/JEV.V2I0.20360 [retrieved on 2013-05-27]

## Description

### FIELD OF THE INVENTION

The present invention relates to a method for determining the level of CNS-derived materials (biomarkers) by measuring the biomarker level in the CNS-derived exosomes enriched from the biological fluid.

### STATEMENT OF U.S. GOVERNMENT INTEREST

The work described herein was performed under grant NS057567 and NS082137 (NINDS Branch) as well as AG033398 (NIA Branch) of the National Institutes of Health (NIH). The U.S. Government may, therefore, have certain rights with respect to the invention pursuant to said grant.

### BACKGROUND OF THE INVENTION

Objective biomarkers are critically needed for assisting with diagnosis, differential diagnosis and or monitoring disease progression of CNS disorders, e.g., Alzheimer's disease (AD), Parkinson's disease (PD), and prion disease. To date, the best performing markers are all based on cerebrospinal fluid (CSF) that is in direct contact with the brain and spinal cord; but obtaining CSF is an invasive procedure that severely limits its routine application. Consequently, peripheral biomarkers (e.g. in blood) of CNS diseases are desperately needed. The challenge, however, is that not all CNS-derived materials can be detected in peripheral body fluids because of tightly regulated various barriers such as blood-brain-barrier. For instance, only a small fraction of proteins seen in human brain/CSF can be readily detected in plasma (Pan et al, J. Proteome Res., 13:4535-4545, 2014). In addition, many CNS-derived materials are also produced by other organ systems. This is indeed one of the fundamental underlying reasons for the fact that, despite decades of research and millions of dollars spent, no peripheral biomarkers have been established for AD, PD or prion disease. Furthermore, none of the current assays of tau and amyloid β (Aβ), the proteins involved in AD, and α-synuclein (a-syn), the critical protein involved in PD, can differentiate the CNS-derived portion from those generated by other systems, e.g. blood components (Barbour et al, Neurodegener Dis., 5(2):55-9, 2008) and muscle (Nagao et al., Muscle & nerve., 22:61-70, 1999), which have much bigger volume masses than the CNS. To state it differently, the signals of CNS diseases are dramatically influenced by peripheral inputs when measured by existing technologies.

US 2010/184046 A1 relates to methods and systems for identifying phenotypes using exosomes and discloses that cell-of-origin exosomes can be used in profiling of physiological states or determining phenotypes.

US 2008/10241924 A1) provides methods and reagents for inducing cell death in tumor cells. The agent for inducing cell death is an antibody against a specific target L1CAM.

M.W. Graner ET AL: "Proteomic and immunologic analyses of brain tumor exosomes", The FASEB Journal, vol. 23, no. 5, 24. December 2008, pages 1541-1557 relates to proteomics and immunologic analysis of brain tumor exosomes and specifically discloses some of the proteins present in brain tumor exosomes

Therefore, there exists a critical need to provide a method to enrich the CNS-derived biomarkers, which is the focus of this invention.

### BRIEF DESCRIPTION OF THE DRAWING

FIG. 1 shows (a) electron micrograph of anti-L1CAM (a nervous system marker)-captured plasma exosomes (inset: immnuogold labeling of L1CAM); (b) Western blot of Alix (a general exosome marker) and L1CAM, by anti-L1CAM capture, or normal mouse IgG capture; (c) α-syn (syn) levels in anti-L1CAM-captured plasma exosomes, compared to the levels in normal mouse IgG-captured (mIgG) or "Empty" (no bead "capture") samples; (d) α-syn (syn) levels using exosome-poor plasma vs. whole plasma.
FIG. 2 shows the evaluation of α-syn concentrations in clinical samples. α-Syn concentrations were measured using Luminex and comparisons were performed for α-syn in L1CAM-containing exosomes isolated from plasma (a), or total α-syn in plasma (b).

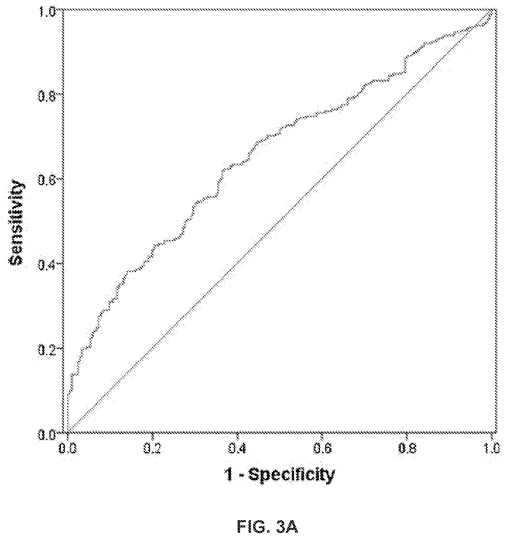

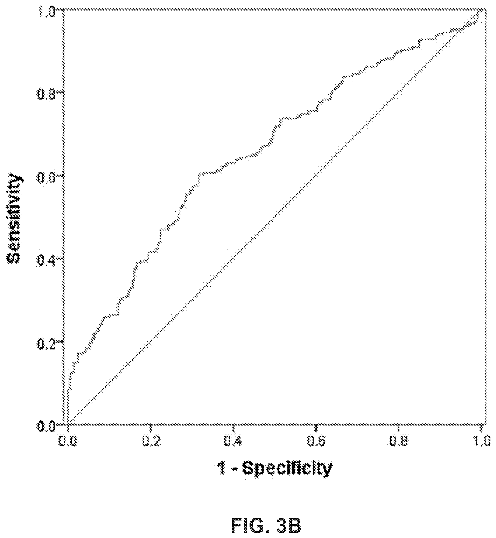

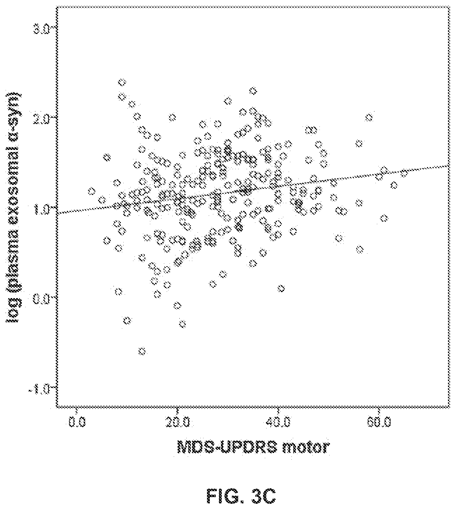

FIGs. 3A and 3B shows the ROC (Receiver Operating Characteristics) analysis of α-syn for PD diagnosis. FIG 3A shows that in the whole cohort (267 patients with PD and 215 healthy controls), the plasma exosomal α-syn provided an AUC (Area Under Curve) of 0.654 (sensitivity=70.1%, specificity=52.9%) for PD versus controls. FIG 3B shows that in a subset of subjects with CSF samples available (100 PD and 100 controls), the CSF total α-syn provided an AUC of 0.724 (sensitivity=76.8%, specificity=53.5%), i.e. the performance of L1CAM enriched exosome marker (a-syn) is as good as what is provided by measuring CSF α-syn. FIG. 3C shows a significant correlation between the plasma exosomal α-syn and the disease severity, indexed by the UPDRS (Unified Parkinson's Disease Rating Scale) motor score, was observed in PD patients (r=0.176, p=0.004, Pearson correlation).
FIGs. 4A and 4B show the levels of radioactivity in the brain (FIG. 4A) and the plasma (FIG. 4B) at 2, 5, 10, 20 and 60 min after injection of ¹²⁵I-labeled tau into intracerebroventricule.
FIG. 4C shows radioactive Tau (cpm/µL plasma) in exosome fractions (exo) and in supernatant fractions (sup). Data shown are mean ± SD from 5 mice. CPM: counts per minute.

### DETAILED DESCRIPTION OF THE INVENTION

### Definition

"A binding pair," as used herein, refers to two molecules that are attracted to each other and specifically bind to each other. Examples of binding pairs include, but not limited to, an antigen and an antibody against the antigen, a ligand and its receptor, complementary strands of nucleic acids, biotin and avidin, biotin and streptavidin, lectin and carbohydrates. Preferred binding pairs are biotin and streptavidin, biotin and avidin, fluorescein and anti-fluorescein, digioxigenin/anti-digioxigenin.

"CNS-derived exosomes," as used herein, refer to exosomes containing materials (such as protein and nucleic acids) derived from the CNS, i.e., brain and/or spinal cord.

"Exosomes," as used herein, are 40-100nm extracellular vesicles that are released from a multitude of cell types, and perform diverse cellular functions including intercellular communication, antigen presentation, and transfer of proteins as well as nucleic acids, e.g. mRNA and miRNA.

"Immobilized," as used herein, refers to reagents being fixed to a solid surface. When a reagent is immobilized to a solid surface, it is either be non-covalently bound or covalently bound to the surface.

"L1CAM," as used herein, refers to a cell adhesion molecule expressed by nervous system. L1CAM is a transmembrane protein; it is a neuronal cell adhesion molecule, member of the L1 protein family, of 200-220 kDa, and involved in axon guidance in addition to cell migration with an implication in treatment-resistant cancers (cancer cells are not relevant in this setting). L1CAM has also been designated CD171 (cluster of differentiation 171).

The inventor has discovered that CNS-derived exosomes may cross multiple layers of the blood-brain barrier, by the mechanisms yet to be defined. The inventor has discovered that CNS-derived exosomes, which carry unique, disease-specific biomarkers, can be detected in vivo in blood and other peripheral body fluids.

Disclosed herein is a method for isolating and enriching exosomes derived from the CNS in a biological fluid such as blood, serum, plasma, saliva, or urine. The inventor has also discovered that CNS-derived neurological biomarkers can be detected and/or quantitated from the enriched exosomes derived from the CNS in a biological fluid, and the results are useful for detecting a neurological disease such as AD, PD, and prion disease. It is also possible to use these peripheral body fluid based but CNS specific markers for differential diagnosis, monitoring disease progression and or objectively assessing treatment effects of CNS diseases.

Disclosed herein is a method for enriching CNS-derived exosomes from a biological fluid of a subject. The method comprises the steps of: (a) contacting a biological fluid containing CNS-derived exosomes with an anti-L1CAM antibody to form an immunocomplex; (b) binding CNS-derived exosomes in the biological fluid to a solid phase through the immunocomplex; and (c) separating the solid phase bound exosomes from the biological fluid to enrich the CNS-derived exosomes. In one embodiment, the anti-L1CAM antibody in step (a) is immobilized on the solid phase. The biological fluids suitable for this invention include blood, serum, plasma, saliva, and urine. A preferred biological fluid is blood, serum, plasma, or saliva.

To specifically detect a neurological disorder in a biological fluid, the present invention uses an immunoaffinity capturing protocol to isolate LICAM-containing exosomes from a biological fluid of a subject. L1CAM is a marker on the surface of exosomes derived from the CNS (Simpson et al., Journal of Extracellular Vesicles 1:18374, 2012). L1CAM is specifically expressed by nervous system; it has no expression in other organ systems (reported to date), including blood cells and platelets. For instance, Anti-L1CAM antibody is specific and does not bind to NK and NKT cells of the immune system like NCAM does (Fiandaca et al., Alzheimer's Dementia, S1552-5260(14)02469-8, 2014); and thus does not capture exosomes derived from immune cells or other organ systems non-specifically.

The anti-L1CAM antibody used to capture the CNS-derived exosomes can be a polyclonal antibody, a monoclonal antibody, single chain antibody, or an antibody fragment containing the L1CAM antigen binding domain such as Fab or F(ab')₂ fragment.

The anti-L1CAM antibody can be immobilized on a solid phase, or it can be in a liquid phase when contacting CNS-derived exosomes in a biological fluid to form an immunocomplex, and then the anti-L1CAM bound exosomes are bound to a solid phase immobilized with reagents that can capture anti-L1CAM.

In one preferred embodiment, the anti-L1CAM is bound to a solid phase when contacting a biological fluid. Methods to immobilize reagents to the solid phase are common in immunochemistry and involve formation of covalent, hydrophobic or electrostatic bonds between the solid phase and reagent. Anti-L1CAM can be directly immobilized on a solid phase. Alternatively, anti-L1CAM can be indirectly immobilized on a solid phase through a binding pair. For example, a first member of a binding pair (e.g., streptavidin, anti-fluorescein, etc.) can be first immobilized either by adsorption to a solid surface or by covalently binding to aminopropylsilane coated on a solid surface. Then anti-L1CAM that is labeled with a second member of a binding pair (e.g., biotin, fluorescein, etc.) can be bound to the solid surface through the binding of biotin-streptavidin or fluorescein and anti-fluorescein (a binding pair).

After the CNS-derived exosomes are specifically captured by a solid phase through the immunocomplex of L1CAM and anti-LlCAM, the exosomes are separated from the biological fluid to enrich the CNS-derived exosomes. The solid phase bound exosomes can be used directly or they can be eluted from the solid phase for further use and/or measurement.

The L1CAM-containing exosomes in the biological fluid produced by the immuno-capture method of the present invention, are shown to have similar quality, as demonstrated by electron microscopy or other measurement, to those obtained by other exosome isolation techniques, including the current "gold standard" - ultracentrifugation plus density gradient. Since no ultracentrifugation is involved in our protocol, the method of the present invention is more practical for use in a routine clinical setting after further optimization. In addition, ultracentrifugation does not provide the specificity of exosomes only derived from the CNS.

The inventor has discovered that measurements of biomarkers contained in CNS-derived exosomes in a biological fluid are useful in detecting neurological diseases. The biomarkers can be either protein or nucleic acids such as DNA or RNA. For example, α-syn or phosphorylated α-syn (e.g., serine 129-α-syn, the phosphorylation of α-syn at residue serine-129 or ps129) in CNS-derived exosomes are biomarkers for PD. Tau or phosphorylated tau, e.g. p-tau 181, in CNS-derived exosomes is a biomarker of AD. Prion protein or phosphorylated prior protein in CNS-derived exosomes is a biomarker of prion disease such as Creutzfeldt-Jakob Disease.

RNAs contained in CNS-derived exosomes as biomarkers for AD include those associated with genes UBAP2L, YBX1, SERF2, UBE2B, RPL10A, H3F3AP4, PPP2CA, NMD3, RNF7, RPLP0, SPARC, WTAP, HNRNPU, LINC00265, INMT, SLC35E2, CT60, SYNCRIP, RGS2, SMC6, ARSA, SPDYE7P, SMIM17, TRAF3IP2-AS1, KCNC2, SLC24A1, HLCS, GOSR1, MN1, MGAT5, NBPF14, FBXO31, WDR52, TBC1D2B, ZNF648, NBPF16, PAGR1, AQP2, PRKCI, SCN2B, DPYSL3, TMEM26, TSPAN11, ELL2, FAM186A, CD59, THSD4, GOLGA6B, ARHGEF5, PKD1, BPTF, FLG, POM121L10P, NXPH3, H3F3A, SH3TC2, GGCX, and GREB1.

RNA contained in CNS-derived exosomes as biomarkers for PD include those associated with genes BLOC1S1, UBE2L3, RNF149, FAM89B, LCE6A, NT5DC2, PPP1CC, CCL5, HDLBP, HNRNPAB, NXN, SLC9A4, EIF2AK1, PAPOLA, TRIM50, SIX4, RAB3IP, VANGL2, DHRSX, FOXP4, SYNM, ZNF543, ATF6, LOC100132832, and BLOC1S1-RDH5.

Protein biomarkers can be measured in enriched exosomes while captured on a solid phase, or after eluted from the solid phase. For a protein biomarker that is exposed on the surface of exosomes, it can be measured without lysis of the exosomes. For a protein biomarker that is contained within the exosomes, it can be measured after lysis of the exosomes. Protein biomarkers can be measured by any method known to a person skilled in the art. Immunoassays such as ELISA, Luminex, and more recently Quanterix are preferred methods for measuring protein biomarkers.

For nucleic acid biomarkers, the captured exosomes need to be lysed before the nucleic acid biomarkers are measured. Nucleic acids can be detected by any method known to a person skilled in the art, e.g., DNA or RNA probe, or any known sequencing techniques.

The present invention is directed to method for detecting a neurological disease or for differential diagnosis in a subject. The method comprises the steps of: (a) contacting a biological fluid from a subject with an anti-L1CAM antibody to form an immunocomplex, wherein the biological fluid is blood, serum, plasma, saliva, or urine; (b) binding CNS-derived exosomes in the biological fluid to a solid phase through the immunocomplex; (c) separating the solid phase bound exosomes from the biological fluid to enrich the CNS-derived exosomes, (d) determining the level of a biomarker from the enriched CNS-derived exosomes, wherein an elevated level of the biomarker from the CNS-derived exosomes in the subject comparing with a control level from a normal subject (for detecting) or from a subject with another neurological disease (for differential diagnosis) indicates that the subject has the neurological disease (e.g. AD, PD or prion disease). In one embodiment, the biomarker is α-syn or phosphorylated α-syn and the neurological disease is PD. In another embodiment, the biomarker is tau, phosphorylated tau or Aβ species and the neurological disease is AD. In another embodiment, the biomarker is Prion protein and the neurological disease is prion disease.

The present invention is further directed to a method for monitoring the progression of a neurological disease in a subject. The method comprises the steps of: (a) obtaining biological fluid samples at different time points (e.g., at time zero, 6 months, 1 year, or 2 years) from a subject, wherein the biological fluid is blood, serum, plasma, saliva, or urine; (b) contacting each sample with an anti-L1CAM antibody to form an immunocomplex, (c) binding CNS-derived exosomes in each biological fluid to a solid phase through the immunocomplex; (d) separating the solid phase bound exosomes from each sample to enrich the CNS-derived exosomes, (e) determining the level of a biomarker in the enriched CNS-derived exosomes from each sample, wherein an elevated level in the sample of a later time point indicates that the disease is progressive. In one embodiment, the biomarker is α -syn or phosphorylated α -syn and the neurological disease is PD. In another embodiment, the biomarker is tau or phosphorylated tau or Aβ species and the neurological disease is AD. In another embodiment, the biomarker is Prion protein or phosphorylated prion protein and the neurological disease is prion disease.

The present invention is further directed to a method for monitoring a drug treatment of a neurological disease in a subject. The method comprises the steps of: (a) obtaining biological fluid samples before and after drug treatment from a subject, wherein the biological fluid is blood, serum, plasma, saliva, or urine; (b) contacting each sample with an anti-L1CAM antibody to form an immunocomplex, (c) binding CNS-derived exosomes in each biological fluid to a solid phase through the immunocomplex; (d) separating the solid phase bound exosomes from each sample to enrich the CNS-derived exosomes, (e) determining the level of a biomarker in the enriched CNS-derived exosomes from each sample, wherein a decreased level in the sample after drug treatment indicates that the drug treatment is effective. In one embodiment, the biomarker is α-syn or phosphorylated α-syn and the neurological disease is PD. In another embodiment, the biomarker is tau or phosphorylated tau or Aβ species and the neurological disease is AD. In another embodiment, the biomarker is Prion protein or phosphorylated prion protein and the neurological disease is prion disease.

The subject of the present invention is a mammal subject such as a human, horse, and dog; with human being the preferred subject.

The following examples further illustrate the present invention. These examples are intended merely to be illustrative of the present invention and are not to be construed as being limiting.

### EXAMPLES

### Example 1. Exosome isolation

Exosomes were isolated from mouse or human plasma using antibody-coated superparamagnetic microbeads following a protocol adapted from Tauro et al (Methods 56: 293-304, 2012). Briefly, 10 µg of anti-L1CAM antibodies (clone UJ127, Abcam, Cambridge, MA, USA), or anti-CD63 antibodies (clone H5C6, BD Biosciences, San Diego, CA, USA), or normal mouse IgGs (Santa Cruz Biotechnology, Dallas, TX, USA) as negative controls, were coated onto one set (1 mg) of M-270 Epoxy beads using a DYNABEADS® Antibody Coupling Kit (Life Technologies, Grand Island, NY, USA) according to the manufacturer's instructions. After thawing quickly (within 2 minutes) at 37°C, plasma samples (>300 µL) were centrifuged at 2,000 × g for 15 minutes followed by 12,000 × g for 30 minutes, and then the supernatant was diluted 1:3 with phosphate buffered saline (PBS) (pH7.4). One set of antibody-coated beads and 900 µL of diluted plasma were incubated for about 24 hours at 4°C with gentle rotation. The beads were then washed four times with 1 mL of 0.1% bovine serum albumin (BSA)/PBS (pH7.4) and transferred into a new tube.

Exosomes were eluted from the beads with 60 µL of a 1:1 mixture of 0.1 % BSA/PBS (pH7.4) and a fixing buffer (4% paraformaldehyde/5% glutaraldehyde) for electron microscopy imaging. Or exosomes were lysed by incubating the beads in 110 µL of 1% Triton X-100 plus 10% of a protease inhibitor cocktail (P2714, Sigma-Aldrich, St Louis, MO, USA; prepared in 10 ml of H₂O) in 0.1% BSA/PBS (pH7.4) for 1 hour at room temperature with gentle shaking for Luminex measurements and other analyses.

Exosomes in clinical plasma samples were extracted in batches, and PD and control samples were distributed into each batch. Two reference plasma samples, pooled from 30 healthy controls, were added into each batch to help to eliminate batch variations.

For quality control and assay development, some reference plasma samples (pooled from healthy old controls) were also diluted in PBS and then subjected to ultracentrifugation (100,000 x g for 3 hours at 4°C); the pellet was then resuspended, loaded onto an OptiPrep™ density gradient, and centrifuged at 100,000 × g for 18 hours at 4°C to obtain exosomes as described in Tauro et al (supra). Exosome-poor plasma samples were prepared by removing exosomes after a 2-step ultracentrifugation (180,000 × g for 3 hours at 4°C × 2).

### Example 2. Characterization of anti-L1CAM-captured exosomes from human blood plasma

### Luminex Assays

Exosome preparations of 100 µL (extracted from 300 µL of plasma) were used to quantify α-syn with an established Luminex protocol (Brain 133:713-726, 2010).

### Electron microscopy

Isolated exosome preparations mixed 1:1 with 4% (v/v) paraformaldehyde and 5% (v/v) glutaraldehyde were layered onto formvar/carbon-coated 300 mesh copper grids (Polysciences, Warrington, PA, USA), and allowed to dry for 20 minutes at room temperature. For direct imaging, grids were then washed twice with water for two minutes, and stained with 2% (w/v) uranyl acetate in water (Electron Microscopy Sciences, Hatfield, PA, USA) for 20 minutes at room temperature. For immunogold staining, grids were washed with PBS before blocking for 30 minutes using a 1%BSA/5% normal goat serum/PBS buffer. The grids were re-washed with PBS, incubated with or without a 1:50 dilution of the anti-L1CAM antibody (abcam) in blocking buffer for two hours at room temperature, and then incubated with an 18-nm gold conjugated goat anti-mouse IgG antibody (abcam) for 60 minutes at room temperature. The grids were washed again and left at room temperature to dry before contrasting with uranyl acetate. Imaging was performed on a JEOL (Peabody, MA, USA) 1230 transmission electron microscope.

### Western blot analysis

Western blotting was performed following a standard protocol. Exosome samples (∼10 µg proteins) were solubilized with Laemmli sample buffer and separated on a ID SDS-PAGE gel before transfer to a polyvinylidene difluoride membrane. The membrane was probed with the following primary antibodies: mouse anti-human L1CAM (Abcam, 1:500) and mouse anti-human Alix (Cat# ABC40, Millipore, Billerica, MA, USA; 1:1000).

To examine the expression of L1CAM in human blood cells, cell lysates (100 µg proteins) from red blood cells and platelets were used, along with human cerebral cortex (100 µg proteins) homogenates as a control; membranes were probed with the anti-L1 CAM antibody.

### Results

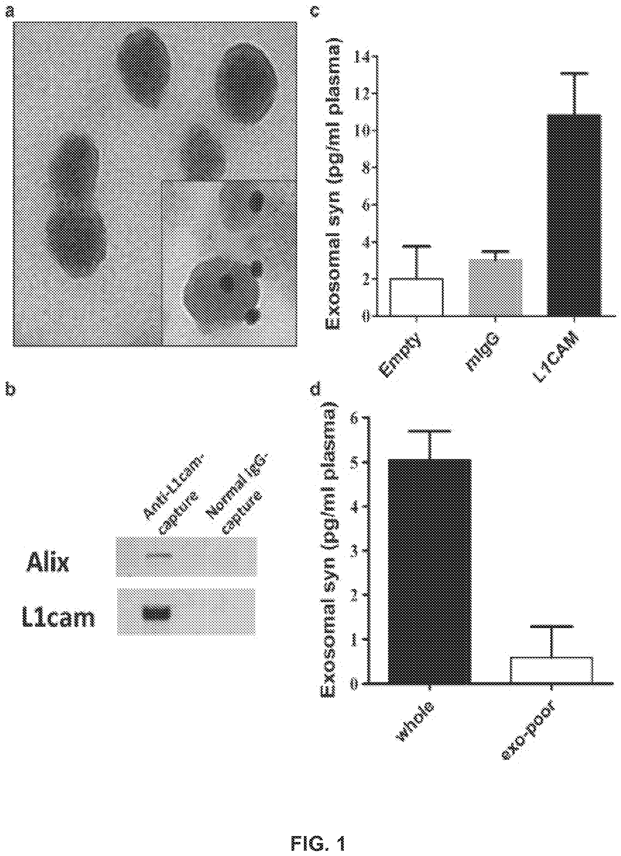

FIG. 1(a) shows the electron micrograph of anti-L1CAM-captured plasma exosomes (inset: immnuogold labeling of L1CAM). The Western blot of FIG. 1(b) shows that Alix, a common exosome marker, and L1CAM were enriched with anti-L1CAM capture, but not with normal IgG capture.

FIG. 1 (c) shows that α-syn levels in anti-L1CAM-captured plasma exosomes were higher than the levels in normal mouse IgG-captured (mIgG) plasma exosomes or "Empty capture" (no bead) plasma exosome. The α-syn signal in anti-L1CAM-captured plasma exosomes was unlikely to be from free α-syn contamination, because the signal from normal mouse IgG-captured or "empty capture" samples was minimal.

To further confirm that the α-syn signal was from exosomes, exosome-poor plasma was generated using ultracentrifugation. Compared to regular plasma, the α-syn signal in L1CAM-containing exosomes in this exosome-poor plasma was reduced more than 10-fold (FIG. 1d).

The L1CAM expression in blood cells was also examined, and the results show that L1CAM was not detectable in red blood cells and platelets.

### Example 3. Evaluation of plasma exosomal α-syn in clinical samples

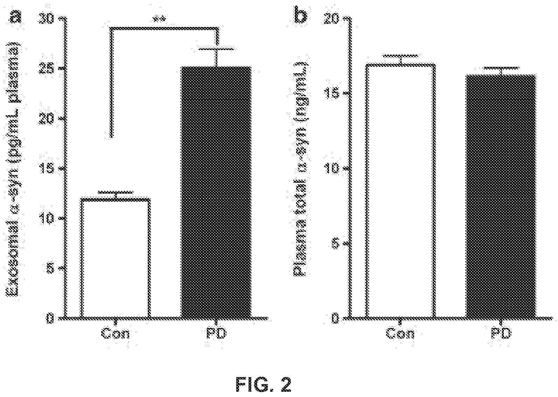

A large cohort of 267 PD and 215 age- and sex- matched healthy control plasma samples are collected and their α-syn concentrations in plasma LICAM-containing exosomes and in whole plasma were measured using Luminex assays (see Example 1). The results are shown in FIG. 2. FIG. 2a shows that the α-syn concentrations in plasma LICAM-containing exosomes were significantly higher in patients with PD compared to healthy controls (*p*<0.0001).
FIG. 2b shows no significant difference in the total plasma α-syn concentrations in PD versus controls (p=0.134, t-test).

To further evaluate the potential for plasma α-syn in L1CAM-containing exosomes to aid in PD diagnosis, ROC (receiver operating characteristic) curves for analytes were generated in 267 patients with PD and 215 healthy control, to characterize their sensitivity and specificity in distinguishing PD from healthy control subjects. The performance of plasma exosomal α-syn was found to be moderate (AUC = 0.654, sensitivity = 70.1%, specificity = 52.9%) (FIG. 3A). ROC analysis was also performed in a subset of subjects with CSF samples available (100 PD and 100 controls); FIG. 3B shows that the CSF total α-syn provided an AUC of 0.724 (sensitivity=76.8%, specificity=53.5%). The above results demonstrate essentially equivalent diagnostic performance (sensitivity and specificity) has been achieved comparing plasma exosomal α-syn to CSF total α-syn, which is the best molecular biomarker of PD described to date (Mollenhauer et al., Exp Neurol 213 (2):315-325, 2008).

Furthermore, the correlation between plasma exosomal α-syn and PD disease severity was examined. Significant correlations between the plasma α-syn in L1CAM-containing exosomes (r=0.176, *p*=0.004, Pearson correlation; see FIG. 3C) or the plasma exo/total α-syn ratio (r=0.130, p=0.035) with the UPDRS motor scores were observed. The results show that plasma exosomal α-syn displayed a significant, though weak, correlation with disease severity (UPDRS motor), indicating that plasma exosomal α-syn is useful in monitoring or predicting disease progression. To this end, it should be emphasized that this correlation is not observed in CSF α-syn in relationship to PD severity by several groups (e.g., Hong et al, Brain, 133:713-26, 2010). Therefore, the plasma exosomal α-syn performs better than CSF α-syn in correlating with PD severity and also likely in correlating with PD progression.

### Example 4. Measurement of tau in anti-L1CAM enriched exosomes.

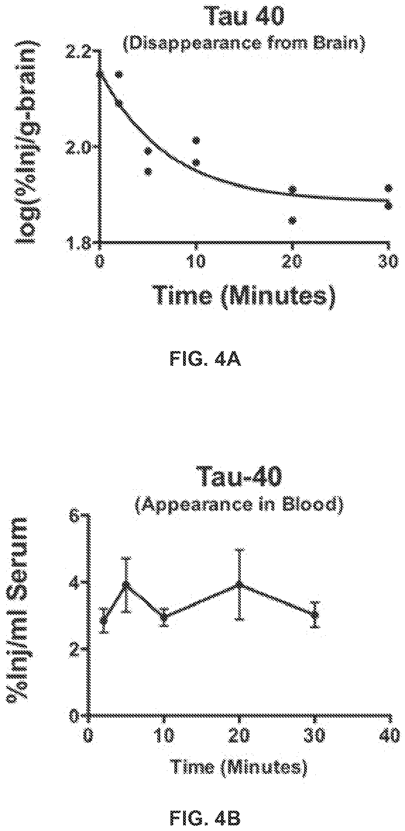

Mice were intracerebroventricularly injected with ¹²⁵I-labeled tau 2N4R (I-Tau). Brain and blood plasma were then collected at 2, 5, 10, 20 and 60 min after injection. Levels of radioactivity in the brain (FIG. 4A) and the plasma (FIG. 4B) were determined using a gamma counter. The results show that tau was transported from the brain to blood.

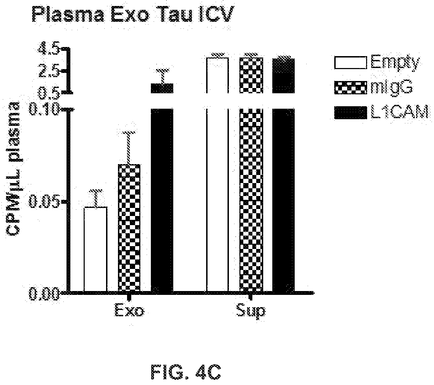

Blood was collected at 60 min after injection, followed by L1CAM-containing exosome extraction from platelet-free plasma (see Example 1). Levels of radioactivity were measured in the whole plasma, the exosome fraction (Exo), and the exosome-less fraction (supernatant after immunoaffinity capture). The results of radioactive Tau (cpm/µL plasma) are shown in exosome fractions and in supernatant fractions (FIG. 4C). The results show that tau, the key marker for AD, is well detectable in exosome enriched by anti-L1CAM over control exosomes (empty exosomes or mouse-IgG attached exosomes). Tau species are also readily detectable in human blood (data not shown).

### Example 5. L1CAM detected in human saliva

Human saliva from normal subject was collected according to the procedures described by Devic et al (Brain. 134(Pt 7):e178. doi: 10.1093/brain/awr015. Epub 2011 Feb 24). Briefly, saliva samples were collected between 9-11 AM from individuals without pre-existing health concerns. To remove insoluble materials and debris, the samples were centrifuged at 15,000 g for 15 min at 4°C. 10% (v/v) protease inhibitor cocktail was immediately added to the supernatant and then the proteins were precipitated with 20% (v/v) of TCA on ice for 1 hour. The mixture was centrifuged at 15,000 g for 15 min at 4°C. The supernatant was removed and the pellet was washed with ice-cold acetone twice. The protein concentration was assessed with a BCA protein assay kit (Thermo Scientific Pierce), employing BSA as a standard and then the sample was stored at -20°C until analysis. The supernatant was incubated with anti-L1CAM antibody coated beads or normal mouse IgG coated beads (negative control) according to the protocols described in Example 1.

The captured exosomes were lysed for Western blot analysis according to the protocols described in Example 2. The membrane was probed with mouse anti-human Alix. Alix (a common exosome marker) was clearly detectable with an expected molecular weight of 95 kilodalton (kDa) in exosomes captured by anti-L1CAM, but not detectable in exosomes captured by normal mouse IgG (negative control). There are no other nonspecific bands except known heavy and light chains of human IgG with appropriate molecular weights of 50 kDa and 25 kDa, respectively.

In anti-L1CAM captured exosomes, α-syn was also detectable by Luminex method as described in Example 3 but optimized for α-syn assays in human saliva (Devic et al Brain 2011 Jul;134(Pt 7):e178.doi 10.1093/brain/awr015. Epub 2011 Feb 24).

### Example 6. Nucleic Acids extracted from L1CAM exosomes

To isolate CNS-derived nucleic acids, L1CAM-exosomes were enriched from the plasma of a total of 72 subjects (24 with AD, 24 with PD and 24 age-matched health controls), according to the methods described in Example 1. Each category (AD, PD and Health Controls) was divided into three small pools (n=8/group) before RNAseq analysis.

Nucleic acids were extracted from L1CAM exosomes. Following the single cell RNA seq protocol (Tang et al, Nat. Protoc. 5, 516-535, 2010), the extracted RNA was reverse transcribed to cDNA. These cDNAs were pre-amplified to about 50-80 nanograms with universal primers.

After cDNA fragmentation with Covaris S2 system, the fragmented cDNA was applied to library construct with DNA library construction kit (NEB #E7370L).

By comparing the genes identified in the 3 pools, there are 58 genes uniquely identified in AD samples, but not in PD or healthy control samples. Those 58 genes are: UBAP2L, YBX1, SERF2, UBE2B, RPL10A, H3F3AP4, PPP2CA, NMD3, RNF7, RPLP0, SPARC, WTAP, HNRNPU, LINC00265, INMT, SLC35E2, CT60, SYNCRIP, RGS2, PSMC6, ARSA, SPDYE7P, SMIM17, TRAF3IP2-AS1, KCNC2, SLC24A1, HLCS, GOSR1, MN1, MGAT5, NBPF14, FBXO31, WDR52, TBC1D2B, ZNF648, NBPF16, PAGR1, AQP2, PRKCI, SCN2B, DPYSL3, TMEM26, TSPAN11, ELL2, FAM186A, CD59, THSD4, GOLGA6B, ARHGEF5, PKD1, BPTF, FLG, POM121L10P, NXPH3, H3F3A, SH3TC2, GGCX, and GREB1.

There are 25 genes uniquely identified in PD samples, but not in AD or healthy control samples. Those 25 genes are BLOC1S1, UBE2L3, RNF149, FAM89B, LCE6A, NT5DC2, PPP1CC, CCL5, HDLBP, HNRNPAB, NXN, SLC9A4, EIF2AK1, PAPOLA, TRIM50, SIX4, RAB3IP, VANGL2, DHRSX, FOXP4, SYNM, ZNF543, ATF6, LOC100132832, and BLOC1S1-RDH5.

It is to be understood that the foregoing describes preferred embodiments of the present invention and that modifications may be made therein without departing from the scope of the present invention as set forth in the claims.

## Claims

1. A method for detecting a neurological disease in a subject, comprising the steps of:
(a) contacting a biological fluid from a subject with an anti-L1CAM antibody to form an immunocomplex, wherein the biological fluid is blood, serum, plasma, or saliva;
(b) binding CNS-derived exosomes in the biological fluid to a solid phase through the immunocomplex;
(c) separating the solid phase bound exosomes from the biological fluid to enrich the CNS-derived exosomes, and
(d) determining the level of a biomarker from the enriched CNS-derived exosomes,
wherein an elevated level of the biomarker from the CNS-derived exosomes in the subject comparing with a control level from a subject without the neurological disease indicates that the subject has the neurological disease,
wherein the neurological disease is Parkinson's disease, Alzheimer's disease or prion disease.

2. The method according to Claim 1, wherein the neurological diseases is Parkinson's disease, and the biomarker is α-synuclein or phosphorylated α-synuclein.

3. The method according to Claim 1, wherein the neurological diseases is Alzheimer's disease, and the biomarker is tau or phosphorylated tau.

4. The method according to Claim 1, wherein the neurological diseases is prion disease, and the biomarker is prion protein.

5. The method of Claim 1, wherein the anti-L1CAM antibody in step (a) is immobilized on the solid phase.

6. The method of Claim 1, further comprising a step (d) of eluting the bound exosomes from the solid phase.

## Patentansprüche

1. Ein Verfahren zum Erfassen einer neurologischen Erkrankung bei einer Testperson, das folgende Schritte aufweist:
(a) In-Kontakt-Bringen eines biologischen Fluids von einer Testperson mit einem Anti-L1CAM-Antikörper, um einen Immunkomplex zu bilden, wobei das biologische Fluid Blut, Serum, Plasma oder Speichel ist;
(b) Binden von aus dem ZNS stammenden Exosomen in dem biologischen Fluid an eine Festphase durch den Immunkomplex;
(c) Trennen der festphasegebundenen Exosomen von dem biologischen Fluid, um die aus dem ZNS stammenden Exosomen anzureichern, und
(d) Bestimmen der Konzentration eines Biomarkers aus den angereicherten aus dem ZNS stammenden Exosomen,
wobei eine erhöhte Konzentration des Biomarkers aus den aus dem ZNS stammenden Exosomen bei der Testperson im Vergleich zu einer Kontrollkonzentration von einer Testperson ohne die neurologische Erkrankung anzeigt, dass bei der Testperson die neurologische Erkrankung vorliegt,
wobei die neurologische Erkrankung die Parkinson-Krankheit, die Alzheimer-Krankheit oder die Prionenkrankheit ist.

2. Das Verfahren gemäß Anspruch 1, bei dem die neurologische Erkrankung die Parkinson-Krankheit ist und der Biomarker α-Synuclein oder phosphoryliertes α-Synuclein ist.

3. Das Verfahren gemäß Anspruch 1, bei dem die neurologische Erkrankung die Alzheimer-Krankheit ist und der Biomarker Tau oder phosphoryliertes Tau ist.

4. Das Verfahren gemäß Anspruch 1, bei dem die neurologische Erkrankung die Prionenkrankheit ist und der Biomarker das Prionprotein ist.

5. Das Verfahren gemäß Anspruch 1, bei dem der Anti-L1CAM-Antikörper in Schritt (a) auf der Festphase immobilisiert wird.

6. Das Verfahren gemäß Anspruch 1, das ferner einen Schritt (d) eines Eluierens der gebundenen Exosomen aus der Festphase aufweist.

## Revendications

1. Procédé de détection d'une maladie neurologique chez un sujet, comprenant les étapes consistant à:
(a) mettre en contact un fluide biologique d'un sujet avec un anticorps anti-L1CAM pour former un immunocomplexe, où le fluide biologique est du sang, du sérum, du plasma ou de la salive;
(b) lier les exosomes dérivés du SNC dans le fluide biologique à une phase solide par l'intermédiaire de l'immunocomplexe;
(c) séparer les exosomes liés à la phase solide du fluide biologique pour enrichir les exosomes dérivés du SNC, et
(d) déterminer le niveau d'un biomarqueur à partir des exosomes dérivés du SNC enrichis,
dans lequel un haut niveau du biomarqueur à partir des exosomes dérivés du SNC chez le sujet en comparaison avec un niveau de contrôle d'un sujet sans maladie neurologique indique que le sujet présente la maladie neurologique,
dans lequel la maladie neurologique est la maladie de Parkinson, la maladie d'Alzheimer ou la maladie à prions.

2. Procédé selon la revendication 1, dans lequel la maladie neurologique est la maladie de Parkinson et le biomarqueur est l'a-synucléine ou l'a-synucléine phosphorylée.

3. Procédé selon la revendication 1, dans lequel la maladie neurologique est la maladie d'Alzheimer et le biomarqueur est le tau ou le tau phosphorylé.

4. Procédé selon la revendication 1, dans lequel la maladie neurologique est la maladie à prions et le biomarqueur est une protéine à prions.

5. Procédé selon la revendication 1, dans lequel l'anticorps anti-L1CAM à l'étape (a) est immobilisé sur la phase solide.

6. Procédé selon la revendication 1, comprenant par ailleurs une étape (d) d'élution des exosomes liés de la phase solide.
